# EUROPEAN PATENT APPLICATION

(11) **EP 4 471 417 A1**
(43) Date of publication of application: **04.12.2024**
(21) Application number: 23747019.0
(22) Date of filing: 26.01.2023
(51) Int. Cl.: G01N 27/62, H01J 49/04, H01J 49/16

(54) **MASS SPECTROMETRY METHOD FOR FLUOROETHER-BASED COMPOUND**

(30) Priority: 28.01.2022 JP 2022012348
(71) Applicant: DAIKIN INDUSTRIES, LTD., Osaka-shi, Osaka 530-0001 (JP)
(72) Inventor: ARAKAWA, Yuka, Osaka-Shi, Osaka 530-0001 (JP); TANAKA, Yuuji, Osaka-Shi, Osaka 530-0001 (JP); HOSODA, Kazuki, Osaka-Shi, Osaka 530-0001 (JP)
(74) Representative: Hoffmann Eitle
(86) International application number: PCT/JP2023/002400
(87) International publication number: WO 2023/145809

(57) **Abstract**

Provided is a mass spectrometry method for a fluoroether compound, the method comprising (1) preparing a sample solution containing the fluoroether compound and a solvent, (2) ionizing the fluoroether compound in the sample solution and also removing the solvent to produce an ion, and (3) mass-separating the ion to determine the mass of the ion, wherein in the production of the ion, the sample solution is sprayed together with an atomizing gas at 330°C or lower to produce droplets, and the produced droplets are brought into contact with a dry gas at 190°C or lower.

## Description

### TECHNICAL FIELD

The present disclosure relates to a mass spectrometry method for a fluoroether compound.

### BACKGROUND ART

Patent Document 1 discloses a process for analyzing a low molecular weight organic compound having at most 20 carbon atoms in a water/oil repellent composition, the process comprising:
(a) mixing a water/oil repellent composition in which a fluorinated polymer having repeating units based on a compound having a perfluoroalkyl group is dispersed or dissolved in a medium, and a C₁₋₅ alcohol, and agglomerating the fluorinated polymer to obtain a liquid containing agglomerates of the fluorinated polymer,
(b) subjecting the liquid containing the agglomerates of the fluorinated polymer to solid-liquid separation to obtain a liquid phase, and
(c) measuring the concentration of a low molecular weight organic compound having at most 20 carbon atoms in the liquid phase by means of a liquid chromatograph-mass spectrometer, a liquid chromatograph-tandem mass spectrometer, or a gas chromatograph-mass spectrometer.

Non Patent Document 1 discloses that when 2,3,3,3-tetrafluoro-2-(1,1,2,2,3,3,3-heptafluoropropoxy)propanoic acid (HFPO-DA) is analyzed by liquid chromatography-tandem mass spectrometry (LC-MS/MS) using ammonium bicarbonate as a mobile phase additive, fragmentation of HFPO-DA is suppressed, and detection sensitivity for [M-H]⁻ is improved but, on the other hand, the same or a higher degree of a bicarbonate adduct [M+HCO₃]⁻ is also detected. It states that high resolution mass spectrometry (high-resolution MS; HRMS) was carried out using a mass spectrometer manufactured by Waters (product name: Xevo G2-XS QTof) to verify the identity of a bicarbonate adduct [M⁺HCO₃]⁻and fragmentation of HFPO-DA.

### RELATED ART

### PATENT DOCUMENT

Patent Document 1: International Publication No. WO 2009/081822

### NON PATENT DOCUMENT

Non Patent Document 1: "Reduction of LC/MS In-Source Fragmentation of HFPO-DA (GenX) Through Mobile Phase Additive Selection: Experiments to Increase [M-H]- Formation", [online], September 26, 2018, United States Environmental Protection Agency, [searched on November 25, 2021], internet <https://cfpub.epa.gov/si/si_public_record_report.cfm?dirEntryId =342415>

### SUMMARY OF THE INVENTION

### PROBLEM TO BE SOLVED BY THE INVENTION

An object of the present disclosure is to provide a mass spectrometry method capable of highly sensitively detecting a fluoroether compound.

### MEANS FOR SOLVING THE PROBLEM

The present disclosure provides a mass spectrometry method for a fluoroether compound, the method comprising (1) preparing a sample solution containing the fluoroether compound and a solvent, (2) ionizing the fluoroether compound in the sample solution and removing the solvent to produce an ion, and (3) mass-separating the ion to determine the mass of the ion, wherein in the production of the ion, the sample solution is sprayed together with an atomizing gas at 330°C or lower to produce droplets, and the produced droplets are brought into contact with dry gas at 190°C or lower.

### EFFECTS OF INVENTION

The present disclosure can provide a mass spectrometry method capable of highly sensitively detecting a fluoroether compound.

### BRIEF DESCRIPTION OF DRAWINGS

Figure 1 is a chromatogram obtained by liquid chromatography mass spectrometry in Experimental Example 5. The horizontal axis indicates a retention time (min), and the vertical axis indicates a relative peak intensity.
Figure 2 is a chromatogram obtained by liquid chromatography mass spectrometry in Experimental Example 6. The horizontal axis indicates a retention time (min), and the vertical axis indicates a relative peak intensity.

### DESCRIPTION OF EMBODIMENTS

The measurement target of the analysis method described in Patent Document 1 is, for example, perfluorocarboxylic acid such as perfluorooctanoic acid (PFOA). In the case of a fluoroether compound as a measurement target, there is the problem that the fluoroether compound cannot be detected highly sensitively when a conventional mass spectrometry method, the analysis target of which is perfluorocarboxylic acid or the like, is applied as-is.

The measurement target of the analysis method described in Non Patent Document 1 is HFPO-DA, and further improvements to suppress the fragmentation of HFPO-DA and the production of an adduct and to highly sensitively detect [M-H]⁻are desired.

An object of the present disclosure is to provide a mass spectrometry method capable of highly sensitively detecting a fluoroether compound.

Hereinafter, specific embodiments of the present disclosure will now be described in detail, but the present disclosure is not limited to the following embodiments.

The mass spectrometry method for a fluoroether compound according to one embodiment of the present disclosure comprises:
1) preparing a sample solution containing the fluoroether compound and a solvent,
2) ionizing the fluoroether compound in the sample solution and removing the solvent to produce an ion, and
3) mass-separating the ion to determine the mass of the ion.

In step (1), a sample solution containing a fluoroether compound and a solvent is prepared.

The kind of the fluoroether compound is not limited, and the fluoroether compound can be a measurement target of the mass spectrometry method of the present disclosure as long as it is an organic compound having 1 or more fluorine atoms, 1 or more ether bonds, and 2 or more carbon atoms.

Examples of the fluoroether compound include polyoxyfluoroalkylene fluoroalkyl ether, fluoroether carboxylic acid, and fluoroether sulfonic acid.

More specific examples of the fluoroether compound include perfluoroethercarboxylic acid (III) represented by the following general formula (III), perfluoroalkoxyfluorocarboxylic acid (V) represented by the following general formula (V), fluorocarboxylic acid (X) represented by the following general formula (X), alkoxyfluorosulfonic acid (XI) represented by the following general formula (XI), a compound (XII) represented by the following general formula (XII), a compound (XIII) represented by the following general formula (XIII), and a compound (XIV) represented by the following general formula (XIV).

The perfluoroethercarboxylic acid (III) is represented by the following general formula (III):

Rf¹-O-(CF(CF₃)CF₂O)ₙ₃CF(CF₃)COOM (III)

wherein Rf¹ is a perfluoroalkyl group having 1 to 5 carbon atoms; n3 is an integer of 0 to 3; and M is H, a metal atom, NR⁷₄, imidazolium optionally having a substituent, pyridinium optionally having a substituent, or phosphonium optionally having a substituent, wherein R⁷ is H or an organic group.

The alkoxyfluorocarboxylic acid (V) is represented by the following general formula (V):

Rf⁴-O-CY¹Y²-(CF₂)ₘ-COOM (V)

wherein Rf⁴ is a linear or branched partially or fully fluorinated alkyl group or alkenyl group having 1 to 12 carbon atoms and optionally containing an ether bond and/or a chlorine atom; Y¹ and Y² are the same or different and are each H, F, or CF₃; m is 0 or 1; and M is as defined above.

The fluorocarboxylic acid (X) is represented by the following general formula (X):

Rf⁷-O-Rf⁸-O-CF₂-COOM (X)

wherein Rf⁷ is a linear or branched partially or fully fluorinated alkyl group having 1 to 6 carbon atoms and optionally containing an ether bond and/or a chlorine atom; Rf⁸ is a linear or branched partially or fully fluorinated alkyl group having 1 to 6 carbon atoms; and M is as defined above.

The alkoxyfluorosulfonic acid (XI) is represented by the following general formula (XI):

Rf⁹-O-CY²Y²-(CF₂)ₘ-SO₃M (XI)

wherein Rf⁹ is a linear or branched partially or fully fluorinated alkyl group or alkenyl group having 1 to 12 carbon atoms and optionally containing an ether bond and/or a chlorine atom; Y¹ and Y² are the same or different and are each H, F, or CF₃; m is 0 or 1; and M is as defined above.

The compound (XII) is represented by the following general formula (XII): 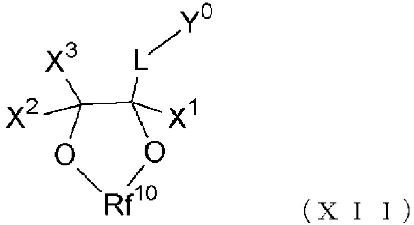 wherein X¹, X², and X³ may be the same or different and are each H, F, or a linear or branched partially or fully fluorinated alkyl group having 1 to 6 carbon atoms and optionally containing an ether bond; Rf¹⁰ is a perfluoroalkylene group having 1 to 3 carbon atoms; L is a linking group; and Y° is an anionic group.

Y⁰ may be -COOM, -SO₂M, or -SO₃M, and may be -SO₃M or COOM, wherein M is as defined above.

Examples of L include a single bond, and a partially or fully fluorinated alkylene group having 1 to 10 carbon atoms and optionally containing an ether bond.

The compound (XIII) is represented by the following general formula (XIII):

Rf¹¹-O-(CF₂CF(CF₃)O)ₙ₉(CF₂O)ₙ₁₀CF₂COOM (XIII)

wherein Rf¹¹ is a fluoroalkyl group having 1 to 5 carbon atoms containing chlorine, n9 is an integer of 0 to 3, n10 is an integer of 0 to 3, and M is as defined above. Examples of the compound (XIII) include CF₂ClO(CF₂CF(CF₃)O)ₙ₉(CF₂O)ₙ₁₀CF₂COONH₄ (a mixture having an average molecular weight of 750, n9 and n10 in the formula are defined above).

The compound (XIV) is represented by the following general formula (XIV):

CXⁱX^{k}=CX^{j}R^{a}-(CZ¹Z²)ₖ-Y⁰ (XIV)

wherein X¹, X^{j}, and X^{k} may be the same or different and are each F, Cl, H, or CF₃; Y⁰ is SO₃M or COOM; R^{a} is a linking group; Z¹ and Z² may be the same or different and are each H, F, or CF₃; k is 0 or 1; and M is defined above.

In particular, a preferable fluoroether compound may be a compound, the highly sensitive detection of which is difficult using conventional mass spectrometry methods, and on which the effect of the mass spectrometry method of the present disclosure is greatly demonstrated, and, for example, compounds represented by the following general formula are preferably used: 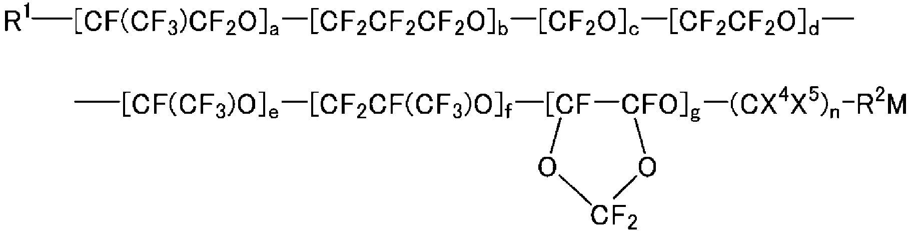 wherein R¹ is H-, F-, Cl-, CH₂=CH-, CH₂=CF-, CF₂=CF-, or CF₂=CFO-; R² is COO or SO₃; a to g may be the same or different and are each an integer of 0 or more, the sum of a to g is an integer of 0 or 1 or more, and when the sum of a to g is 0, R¹ is CF₂=CFO-; X⁴ and X⁵ are each independently H, F, or CF₃; n is an integer of 1 or more; and M is a cation.

In the general formulae above, the respective repeating units may occur in any order, and X⁴ and X⁵ may be the same or different at each occurrence.

a to g may be the same or different, and are each an integer of 0 or more, preferably an integer of 0 to 2, and more preferably 0 or 1.

The sum of a to g is an integer of 0 or 1 or more, preferably an integer of 1 to 5, more preferably an integer of 1 to 3, even more preferably 1 or 2, and particularly preferably 1.

n is preferably an integer of 1 to 3, more preferably 1 or 2, and even more preferably 1.

In one embodiment of the fluoroether compound, g is 0, a to f may be the same or different and are each an integer of 0 or more, and the sum of a to f is an integer of 1 or more.

In one embodiment of the fluoroether compound, g is 0, and a to f may be the same or different and are each an integer of 0 or more, preferably an integer of 0 to 2, and more preferably 0 or 1.

In one embodiment of the fluoroether compound, g is 0, and the sum of a to f is an integer of 1 or more, preferably an integer of 1 to 5, more preferably an integer of 1 to 3, even more preferably 1 or 2, and particularly preferably 1.

In one embodiment of the fluoroether compound, g is 0, and n is preferably an integer of 1 to 3, more preferably 1 or 2, and even more preferably 1.

M is preferably H, a metal atom, NR⁷₄ (R⁷ is H or an organic group), optionally substituted imidazolium, optionally substituted pyridinium, or optionally substituted phosphonium, more preferably H, a metal atom, or NR⁷₄, even more preferably H, an alkali metal (Group 1), an alkaline earth metal (Group 2), or NR⁷₄, and particularly preferably H, Na, K, Li, or NH₄.

In particular, the fluoroether compound is a compound, the highly sensitive detection of which is difficult using conventional mass spectrometry methods, and on which the effect of the mass spectrometry method of the present disclosure is greatly demonstrated, and, therefore, the compounds represented by the following formulae are particularly preferable:

CF₃O(CF₂)₃OCHFCF₂COOM,

C₃F₇OCF(CF₃)CF₂OCF(CF₃)COOM,

CF₃CF₂CF₂OCF(CF₃)COOM,

CF₃CF₂OCF₂CF₂OCF₂COOM,

C₂F₅OCF(CF₃)CF₂OCF(CF₃)COOM,

CF₃OCF(CF₃)CF₂OCF(CF₃)COOM,

CF₂ClCF₂CF₂OCF(CF₃)CF₂OCF₂COOM,

CF₂ClCF₂CF₂OCF₂CF(CF₃)OCF₂COOM,

CF₂ClCF(CF₃)OCF(CF₃)CF₂OCF₂COOM,

CF₂ClCF(CF₃)OCF₂CF(CF₃)OCF₂COOM,

CF₂=CFOCF₂CF(CF₃)OCF₂CF₂COOM,

CF₂=CFOCF₂CF(CF₃)OCF₂CF₂SO₃M,

CF₂=CFOCF₂CF₂SO₃M,

CF₂=CFOCF₂CF₂COOM,

CH₂=CFCF₂OCF(CF₃)COOM, and

CH₂=CFCF₂OCF(CF₃)CF₂OCF(CF₃)COOM

wherein M is H, a metal atom, NR⁷₄, optionally substituted imidazolium, optionally substituted pyridinium, or optionally substituted phosphonium, and R⁷ is H or an organic group.

The solvent is preferably a volatile solvent that can be readily removed, and that can readily produce only the ion that is subject to mass separation, in step (2). Examples of the solvent include solvents commonly used in liquid chromatography mass spectrometry, e.g., alcohols such as methanol, ethanol and isopropyl alcohol, acetonitrile, and water.

In one embodiment, the sample solution is a sample solution, the components of which have been separated by liquid chromatography. When preparing the sample solution using liquid chromatography, step (1) can include a step wherein, for example, a sample containing a fluoroether compound is fed to a column together with a mobile phase, the components are separated while traveling through the column, and the component-separated eluate is discharged from the column as a sample solution.

In one embodiment, component separation using liquid chromatography can be carried out in a liquid chromatography unit (an LC unit) of a liquid chromatograph-mass spectrometer. Such a liquid chromatograph-mass spectrometer includes, for example, an LC unit for separating the components of a sample solution by liquid chromatography, an ionization unit for ionizing the components of the sample solution and removing the solvent, a mass separation unit for separating the ion according to the mass-to-charge ratio, and an ion detection unit for detecting the separated ion.

Examples of liquid chromatography include reverse phase chromatography, normal phase chromatography, hydrophilic interaction chromatography, ion exchange chromatography, and size exclusion chromatography. In one embodiment, reverse phase chromatography is used.

When carrying out liquid chromatography, an isocratic method in which a single mobile phase is used, or a gradient method in which the composition of the mobile phase is continuously changed, can be used. In one embodiment, water and methanol, or water and acetonitrile, are used in the mobile phase, and liquid chromatography is carried out while continuously changing the composition of water and methanol, or the composition of water and acetonitrile, in the mobile phase.

Preferably, the pH of the sample solution is also regulated. The pH of the sample solution may be, for example, less than 8. In one embodiment, the pH is regulated by regulating the pH of the mobile phase fed to the liquid chromatograph such that the pH of the eluate is within a desired range. To regulate the pH of the mobile phase, for example, pH regulators, e.g., acids such as formic acid and acetic acid; ammonium salts such as ammonium formate, ammonium acetate, ammonium bicarbonate, and ammonium hydroxide; and ammonia can be used.

In step (2), an ion is produced by ionizing the fluoroether compound in the sample solution and removing the solvent.

In one embodiment, the ion is produced in the ionization unit of a liquid chromatograph-mass spectrometer. When liquid chromatography is carried out in step (1), the eluate discharged from the column can be introduced into the ionization unit as a sample solution. For example, a method in which ionization is carried out under atmospheric pressure is suitably used for ionization.

An ionization unit compatible with electrospray ionization (ESI), atmospheric pressure chemical ionization (APCI), or the like is used. In one embodiment, electrospray ionization (ESI) is used to produce an ion.

In electrospray ionization, a sample molecule is ionized by producing charged droplets and drying the charged droplets. In the production of droplets, the sample solution is dispersed by electrospray. At this time, production of droplets is facilitated by using an inert gas such as nitrogen as a nebulizer gas. Also, by spraying the sample solution together with an atomizing gas, desolvation from the droplets is facilitated. The atomizing gas may be referred to as sheath gas, desolvation gas, auxiliary gas, heater gas, turbo gas, or the like. Moreover, bringing the droplets into contact with a dry gas further facilitates desolvation and drying of the droplets. The dry gas may be referred to as sweep gas, cone gas, or the like.

In step (2), the sample solution is sprayed together with an atomizing gas at 330°C or lower to produce droplets, the produced droplets are brought into contact with a dry gas at 190°C or lower, and thereby a fluoroether compound can be detected highly sensitively. In conventional mass spectrometry methods, usually the produced droplets are actively vaporized at high temperatures to efficiently evaporate the droplets and, therefore, the significant improvement of detection sensitivity by controlling the atomizing gas and drying gas temperatures to be low is an unexpected, surprising effect.

The temperature of the atomizing gas may be, for example, 100 to 330°C. The temperature of the atomizing gas is preferably 310°C or lower and more preferably 300°C or lower, and is preferably 130°C or higher, more preferably 140°C or higher, and even more preferably 150°C or higher, because the detection sensitivity for the fluoroether compound is further increased.

The temperature of the atomizing gas can be regulated by regulating the parameters referred to as sheath gas temperature, vaporizer temperature, desolvation gas temperature, ESI heater temperature, source temperature, turbo temperature, HSID temperature, and the like.

The temperature of the dry gas may be, for example, 80 to 190°C. The temperature of the dry gas is preferably 175°C or lower, more preferably 165°C or lower, and even more preferably 155°C or lower, because the detection sensitivity for the fluoroether compound is further increased.

The temperature of the drying gas can be regulated by regulating the parameters referred to as ion transfer temperature, source temperature, block heater temperature, desolvation line temperature, source temperature, dry heater temperature, and the like.

Other ionization conditions may be those normally set in measurements involving a liquid chromatograph-mass spectrometer. For example, when using a mass spectrometer manufactured by Agilent, the respective conditions can be set within the following ranges:
Flow rate of atomizing gas: 2 to 12 L/min
Flow rate of dry gas: 3 to 13 L/min
Feeding rate of sample solution: 50 to 1,000 µL/min
Voltage applied when producing ions: 300 to 6,000 V
Nebulizer gas pressure: 30 to 60 psi

In step (3), the ion produced in step (2) is mass-separated to determine the mass of the ion.

In one embodiment, in the mass separation unit and the ion detection unit of a liquid chromatograph-mass spectrometer, the ion is mass-separated according to the mass-to-charge ratio, the separated ion is detected, and thereby the mass of the ion is determined.

The mass separation unit may be a quadrupole mass separation unit, an ion trap mass separation unit, a fan-shaped magnetic field mass separation unit, a time-of-flight mass separation unit, or the like. Also, mass separation units of the same type or mass separation units of different types can be connected and used (a tandem mass spectrometer (MS/MS)). In one embodiment, the mass spectrometry method of the present disclosure is carried out using a liquid chromatograph-tandem mass spectrometer (LC-MS/MS).

According to the mass spectrometry method of the present disclosure, in step (3), an ion represented by the following general formula can be used as a precursor ion: 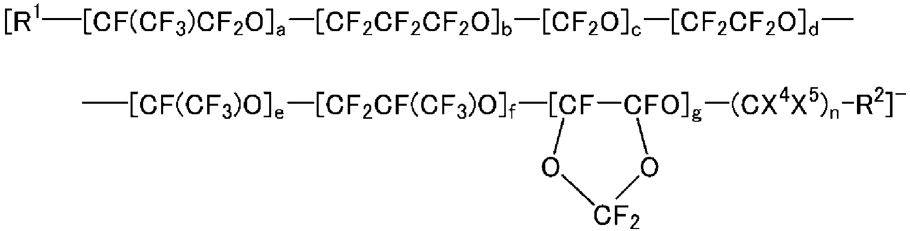 wherein R¹, R², a to g, X⁴, X⁵, and n are as described above; the respective repeating units may occur in any order; and X⁴ and X⁵ may be the same or different at each occurrence. With conventional methods, such an ion cannot be detected highly sensitively (because such an ion shows only a very weak signal) and, therefore, attempts to analyze the mass of a fluoroether compound have been made by detecting a fragment ion instead (e.g., a fragment ion resulting from separation of some atomic group such as COO). However, with such a method, the possibility of a fragment ion resulting from other compounds cannot be ruled out, moreover, it cannot be said that the detection sensitivity is sufficient, and an accurate analysis is thus difficult. According to the mass spectrometry method of the present disclosure, the above ion can be directly detected highly sensitively, and thus the mass of the fluoroether compound can be accurately measured.

In the mass spectrometry method of the present disclosure, the use of a sample solution that contains, in addition to the fluoroether compound and the solvent, a fluorine-containing compound different from the fluoroether compound enables not only the mass of the fluoroether compound but also the mass of the fluorine-containing compound different from the fluoroether compound to be simultaneously determined.

The fluorine-containing compound (the fluorine-containing compound different from the fluoroether compound) that can be included in the targets of the mass spectrometry method of the present disclosure is not limited as long as it is a compound that contains one or more fluorine atoms and that does not contain an ether bond. Examples include:
perfluorocarboxylic acids such as perfluorobutanoic acid, perfluoropentanoic acid, perfluorohexanoic acid, perfluoroheptanoic acid, perfluorooctanoic acid, perfluorononanoic acid, perfluorodecanoic acid, perfluoroundecanoic acid, perfluorododecanoic acid, perfluorotridecanoic acid, and perfluorotetradecanoic acid;
fluorocarboxylic acids resulting from replacement of some fluorine atoms of perfluorocarboxylic acid with hydrogen atoms; perfluorosulfonic acids such as perfluorobutanesulfonic acid, perfluoropentanesulfonic acid, perfluorohexane sulfonic acid, perfluoroheptane sulfonic acid, perfluorooctane sulfonic acid, perfluorononane sulfonic acid, perfluorodecane sulfonic acid, perfluoroundecane sulfonic acid, perfluorododecane sulfonic acid, perfluorotridecane sulfonic acid, and perfluorotetradecane sulfonic acid;
fluorosulfonic acids resulting from replacement of some fluorine atoms of perfluorosulfonic acid with hydrogen atoms, such as 1H,1H,2H,2H-perfluorohexanesulfonic acid, 1H,1H,2H,2H-perfluorooctanesulfonic acid, and 1H,1H,2H,2H-perfluorodecanesulfonic acid; and
derivatives of perfluorocarboxylic acid or perfluorosulfonic acid, such as 2-(N-methylperfluorooctanesulfonamido)acetic acid and 2-(N-ethylperfluorooctanesulfonamido)acetic acid.

While embodiments have been described above, it will be understood that various changes in form and detail can be made without departing from the gist and scope of the claims.
<1> According to the first aspect of the present disclosure, provided is
   a mass spectrometry method for a fluoroether compound, the method comprising:
      1) preparing a sample solution containing the fluoroether compound and a solvent,
      2) ionizing the fluoroether compound in the sample solution and also removing the solvent to produce an ion, and
      3) mass-separating the ion to determine the mass of the ion,
      wherein
   in the production of the ion,
      the sample solution is sprayed together with an atomizing gas at 330°C or lower to produce a droplet, and the produced droplet is brought into contact with a dry gas at 190°C or lower.
<2> According to the second aspect of the present disclosure, provided is
   the mass spectrometry method according to the first aspect, wherein the sample solution has a pH of less than 8.
<3> According to the third aspect of the present disclosure, provided is
   the mass spectrometry method according to the first or second aspect, wherein a pressure when producing the ion is atmospheric pressure.
<4> According to the fourth aspect of the present disclosure, provided is
   the mass spectrometry method according to any one of the first to third aspects, wherein the production of the ion is performed by an electrospray ionization method.
<5> According to the fifth aspect of the present disclosure, provided is
   the mass spectrometry method according to any one of the first to fourth aspects, wherein the fluoroether compound is a compound represented by the general formula: 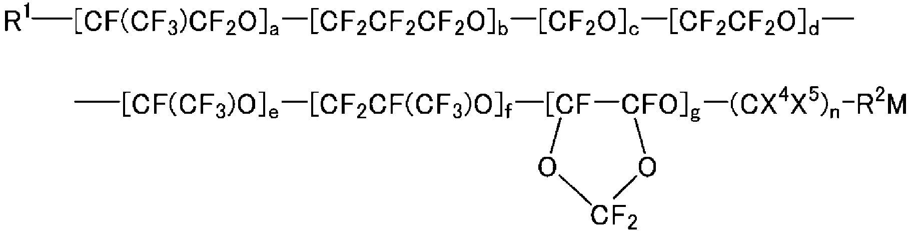 wherein R¹ is H-, F-, Cl-, CH₂=CH-, CH₂=CF-, CF₂=CF-, or CF₂=CFO-; R² is COO or SO₃; a to g may be the same or different and are each an integer of 0 or more, the sum of a to g is an integer of 0 or 1 or more, and when the sum of a to g is 0, R¹ is CF₂=CFO-; X⁴ and X⁵ are each independently H, F, or CF₃; n is an integer of 1 or more; and M is a cation.
<6> According to the sixth aspect of the present disclosure, provided is
   the mass spectrometry method according to the fifth aspect, wherein an ion represented by the following general formula is used as a precursor ion: 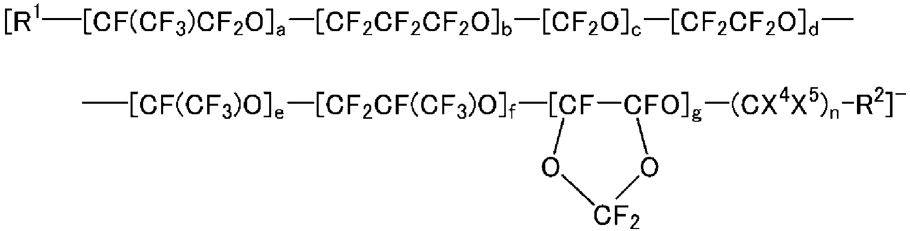 wherein R¹, R², a to g, X⁴, X⁵, and n are as described above.
<7> According to the seventh aspect of the present disclosure, provided is
   the mass spectrometry method according to any one of the first to sixth aspects, comprising preparing a sample solution further containing a fluorine-containing compound different from the fluoroether compound, ionizing the fluorine-containing compound in addition to the fluoroether compound and also removing the solvent to produce the ion of the fluoroether compound and an ion of the fluorine-containing compound, and determining the mass of the ion of the fluorine-containing compound in addition to the mass of the ion of the fluoroether compound.

### EXAMPLES

Next, embodiments of the present disclosure will now be described by way of Examples, but the present disclosure is not limited only to the Examples.

The following apparatuses were used in Experimental Examples 1 to 4.
(Liquid chromatograph-mass spectrometer)
(LC-MS/MS)
LC: 1290 Infinity II manufactured by Agilent
MS: Ultivo LC/TQ manufactured by Agilent

### (LC measurement conditions)

Column: ZORBAX Extend-C18 manufactured by Agilent, 2.1 mm × 50 mm, 1.8 µm
Mobile phase: (Liquid A) 20 mM Aqueous ammonium acetate solution, (Liquid B) LC/MS acetonitrile
Composition: Liquid A:Liquid B = 40:60
Amount of sample introduced: 1 µL
Flow rate: 200 µL/min
Column temperature: 40°C

### (MS measurement conditions)

Ionization method: Negative ESI
<2,3,3,3-Tetrafluoro-2-(1,1,2,2,3,3,3-heptafluoropropoxy)propanoic acid>
MRM monitor ion: 329.0 -> 285.0
Fragmenter voltage: 50 V
Collision energy: 1 V
<PFOA>
MRM monitor ion: 413.0 -> 369.0
Fragmenter voltage: 110 V
Collision energy: 5 V
2,3,3,3-Tetrafluoro-2-(1,1,2,2,3,3,3-heptafluoropropoxy)propanoic acid and PFOA were purchased from FUJIFILM Wako Pure Chemical Corporation.

### Experimental Example 1

Experimental Example 1 demonstrates that an atomizing gas temperature within an extremely limited range significantly increases detection sensitivity for a fluoroether compound.

The sample used (the sample introduced to liquid chromatography) was a 0.1 mass ppm aqueous solution of 2,3,3,3-tetrafluoro-2-(1,1,2,2,3,3,3-heptafluoropropoxy)propanoic acid.

The prepared sample and the above liquid chromatograph-mass spectrometer were used to carry out mass spectrometry on 2,3,3,3-tetrafluoro-2-(1,1,2,2,3,3,3-heptafluoropropoxy)propanoic acid.

The ionization conditions in Experimental Example 1 are as follows:
Temperature of dry gas: 150°C
Flow rate of dry gas: 5 L/min
Temperature of atomizing gas (temperature of sheath gas): As shown in Table 1
Flow rate of atomizing gas: 7.5 L/min
Nebulizer pressure: 50 psi
Frag: 50 V
CE: 1 V
Capillary voltage: As shown in Table 1

The detection sensitivity (response) of each ionization condition was determined from the peak area value of an ion corresponding to the mass of [CF₃CF₂CF₂OCF(CF₃)COO]⁻ in the resulting spectrum. Table 1 shows numerical values calculated based on the detection sensitivity measured under conditions having a dry gas temperature of 150°C, an atomizing gas temperature of 350°C, and a voltage of 1,000 V being 100.

### [Table 1]

**Table 1**

| | | Sheath gas temperature [ °C] | | | | | | | | | |
|---|---|---|---|---|---|---|---|---|---|---|---|
| | | 150 | 220 | 250 | 260 | 300 | 320 | 330 | 340 | 350 | 380 |
| Voltage[V] | 1000 | 120 | 131 | 168 | 141 | 128 | 110 | 107 | 88 | 100 | 55 |
| | 2500 | 168 | 214 | 240 | 226 | 216 | 183 | 182 | 149 | 151 | 110 |
| | 3000 | 165 | 208 | 244 | 222 | 215 | 203 | 187 | 172 | 155 | 112 |
| | 6000 | 144 | 180 | 211 | 196 | 205 | 208 | 197 | 189 | 169 | 135 |

### Experimental Example 2

Experimental Example 2 demonstrates that a drying gas temperature within an extremely limited range significantly increases detection sensitivity for a fluoroether compound.

The sample prepared in Experimental Example 1 and the above liquid chromatograph-mass spectrometer were used to carry out mass spectrometry on 2,3,3,3-tetrafluoro-2-(1,1,2,2,3,3,3-heptafluoropropoxy)propanoic acid.

The ionization conditions in Experimental Example 2 are as follows:
Temperature of dry gas: As shown in Table 2
Flow rate of dry gas: 5 L/min
Temperature of atomizing gas: 250°C
Flow rate of atomizing gas: 7.5 L/min
Nebulizer pressure: 50 psi
Frag: 50 V
CE: 1 V
Capillary voltage: As shown in Table 2

The detection sensitivity (response) of each ionization condition was determined from the peak area value of an ion corresponding to the mass of [CF₃CF₂CF₂OCF(CF₃)COO]⁻ in the resulting spectrum. Table 2 shows numerical values calculated based on the detection sensitivity measured under conditions having a dry gas temperature of 150°C, an atomizing gas temperature of 350°C, and a voltage of 1,000 V being 100.

### [Table 2]

**Table 2**

| | | Dry gas temperature [°C] | | | | | | | | | | | |
|---|---|---|---|---|---|---|---|---|---|---|---|---|---|
| | | 80 | 100 | 150 | 160 | 170 | 180 | 190 | 200 | 210 | 220 | 230 | 250 |
| Voltage[V] | 1000 | 165 | 157 | 168 | 133 | 135 | 118 | 107 | 94 | 54 | 86 | 50 | 36 |
| | 1600 | 255 | 276 | 240 | 191 | 207 | 182 | 159 | 85 | 88 | 94 | 78 | 6 |
| | 2500 | 245 | 282 | 244 | 158 | 216 | 189 | 169 | 69 | 93 | 90 | 80 | 8 |
| | 4000 | 219 | 211 | 232 | 195 | 211 | 194 | 112 | 88 | 89 | 98 | 85 | 60 |
| | 6000 | 220 | 237 | 211 | 204 | 190 | 179 | 163 | 76 | 89 | 94 | 84 | 57 |

### Experimental Examples 3 and 4

Experimental Examples 3 and 4 demonstrate that perfluorooctanoic acid (PFOA) cannot be detected at sufficient sensitivity when the mass of perfluorooctanoic acid (PFOA) is analyzed using the mass spectrometry method of the present disclosure.

The sample used (the sample introduced to liquid chromatography) was a 0.1 mass ppm aqueous solution of perfluorooctanoic acid (PFOA).

The prepared sample and the above liquid chromatograph-mass spectrometer were used to carry out mass spectrometry on perfluorooctanoic acid.

The ionization conditions in Experimental Example 3 are as follows:
Temperature of dry gas: 150°C
Flow rate of dry gas: 5 L/min
Temperature of atomizing gas (temperature of sheath gas): As shown in Table 3
Flow rate of atomizing gas: 7.5 L/min
Nebulizer pressure: 50 psi
Frag: 110 V
CE: 5 V
Capillary voltage: As shown in Table 3

The detection sensitivity (response) of each ionization condition was determined from the peak area value of an ion corresponding to the mass of [CF₃(CF₂)₆COO]⁻ in the resulting spectrum. Table 3 shows numerical values calculated based on the detection sensitivity measured under conditions having a dry gas temperature of 150°C, an atomizing gas temperature of 350°C, and a voltage of 1,000 V being 100.

### [Table 3]

**Table 3**

| | | Sheath gas temperature [°C] | | | |
|---|---|---|---|---|---|
| | | 250 | 340 | 350 | 380 |
| Voltage [V] | 1000 | 75 | 90 | 100 | 103 |

The ionization conditions in Experimental Example 4 are as follows:
Temperature of dry gas: As shown in Table 4
Flow rate of dry gas: 5 L/min
Temperature of atomizing gas: 250°C
Flow rate of atomizing gas: 7.5 L/min
Nebulizer pressure: 50 psi
Frag: 110 V
CE: 5 V
Capillary voltage: As shown in Table 4

The detection sensitivity (response) of each ionization condition was determined from the peak area value of an ion corresponding to the mass of [CF₃(CF₂)₆COO]⁻ in the resulting spectrum. Table 4 shows numerical values calculated based on the detection sensitivity measured under conditions having a dry gas temperature of 150°C, an atomizing gas temperature of 350°C, and a voltage of 1,000 V being 100.

### [Table 4]

**Table 4**

| | | Dry gas temperature [°C] | | | |
|---|---|---|---|---|---|
| | | 100 | 220 | 230 | 250 |
| Voltage [V] | 1000 | 75 | 83 | 78 | 90 |

The following apparatuses were used in Experimental Examples 5 to 6.
(Liquid chromatograph-mass spectrometer)
(LC-MS/MS)
LC: 1290 Infinity II manufactured by Agilent
MS: Ultivo LC/TQ manufactured by Agilent

### (LC measurement conditions)

Column: ZORBAX Extend-C18 manufactured by Agilent, 2.1 mm × 50 mm, 1.8 µm
Mobile phase: (Liquid A) 20 mM Aqueous ammonium acetate solution, (Liquid B) LC/MS acetonitrile

| |
|---|
| Composition: Liquid A:Liquid B = 80:20(0 to 1 min) |
| ↓ (1 to 6 min) |
| 60:40 (6 to 7 min) |
| ↓ (7 to 12 min) |
| 95:5 (12 to 15 min) |

Amount of sample introduced: 5 µL
Flow rate: 300 µL/min
Column temperature: 40°C

### (MS measurement conditions)

Ionization method: Negative ESI

### [Table 5]

**Table 5**

| Peak number | Compound name | MRM monitor ion | Fragmenter voltage | Collision energy |
|---|---|---|---|---|
| 1 | Perfluorobutanoic acid | 213.0→169.0 | 90 V | 5 V |
| 2 | Perfluoropentanoic acid | 263.0→219.0 | 95 V | 5 V |
| 3 | Perfluorohexanoic acid | 313.0→269.0 | 100 V | 5 V |
| 4 | 2,3,3,3-Tetrafluoro-2-(1,1,2,2,3,3,3-heptafluoropropoxy)propanoic acid | 329.0→285.0 | 50 V | 1 V |
| 5 | Perfluoroheptanoic acid | 363.0→319.0 | 105 V | 5 V |
| 6 | Perfluorooctanoic acid | 413.0→369.0 | 110 V | 5 V |
| 7 | Perfluorononanoic acid | 463.0→419.0 | 115 V | 5 V |
| 8 | Perfluorodecanoic acid | 513.0→469.0 | 120 V | 5 V |
| 9 | Perfluoroundecanoic acid | 563.0→519.0 | 125 V | 5 V |
| 10 | Perfluorododecanoic acid | 613.0→569.0 | 130 V | 5 V |
| 11 | Perfluorotridecanoic acid | 663.0→619.0 | 130 V | 5 V |
| 12 | Perfluorotetradecanoic acid | 713.0→669.0 | 130 V | 5 V |

A mixture of perfluorobutanoic acid, perfluoropentanoic acid, perfluorohexanoic acid, perfluoroheptanoic acid, perfluorooctanoic acid, perfluorononanoic acid, perfluorodecanoic acid, perfluoroundecanoic acid, perfluorododecanoic acid, perfluorotridecanoic acid, and perfluorotetradecanoic acid was purchased from Wellington Laboratories.

### Experimental Example 5

Experimental Example 5 demonstrates that 2,3,3,3-tetrafluoro-2-(1,1,2,2,3,3,3-heptafluoropropoxy)propanoic acid can be simultaneously measured with perfluorobutanoic acid, perfluoropentanoic acid, perfluorohexanoic acid, perfluoroheptanoic acid, perfluorooctanoic acid, perfluorononanoic acid, perfluorodecanoic acid, perfluoroundecanoic acid, perfluorododecanoic acid, perfluorotridecanoic acid, perfluorotetradecanoic acid.

The ionization conditions in Experimental Example 5 are as follows:
Temperature of dry gas: 150°C
Flow rate of dry gas: 5 L/min
Temperature of atomizing gas (temperature of sheath gas): 250°C
Flow rate of atomizing gas: 7.5 L/min
Nebulizer pressure: 50 psi
Frag: As shown in Table 5
CE: As shown in Table 5
Capillary voltage: 2,500 V

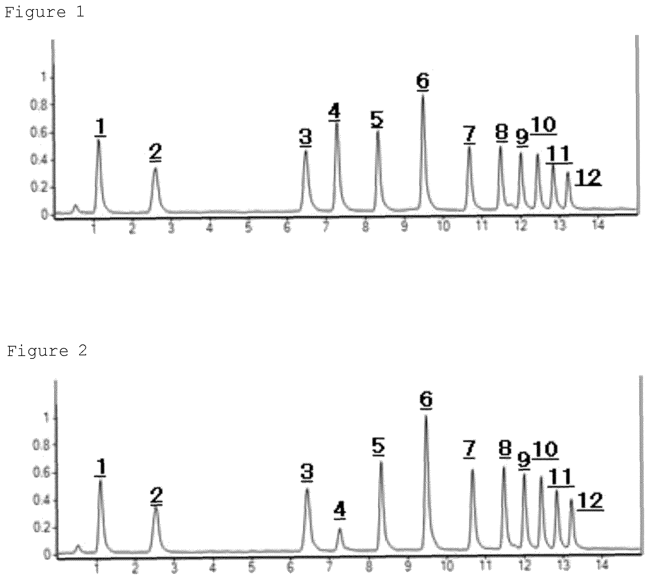

The sample used (the sample introduced to liquid chromatography) was an aqueous solution containing a mixture of 2,3,3,3-tetrafluoro-2-(1,1,2,2,3,3,3-heptafluoropropoxy)propanoic acid with perfluorobutanoic acid, perfluoropentanoic acid, perfluorohexanoic acid, perfluoroheptanoic acid, perfluorooctanoic acid, perfluorononanoic acid, perfluorodecanoic acid, perfluoroundecanoic acid, perfluorododecanoic acid, perfluorotridecanoic acid, and perfluorotetradecanoic acid (the content of each of the 12 compounds was 0.01 mass ppm). The amount introduced was 5 µL. The results of measurement are shown in Figure 1.

### Experimental Example 6

Experimental Example 6 demonstrates that detection sensitivity for a fluoroether compound is lowered at a dry gas temperature outside a limited range in simultaneous measurement of 2,3,3,3-tetrafluoro-2-(1,1,2,2,3,3,3-heptafluoropropoxy)propanoic acid with perfluorobutanoic acid, perfluoropentanoic acid, perfluorohexanoic acid, perfluoroheptanoic acid, perfluorooctanoic acid, perfluorononanoic acid, perfluorodecanoic acid, perfluoroundecanoic acid, perfluorododecanoic acid, perfluorotridecanoic acid, and perfluorotetradecanoic acid.

Measurement was carried out under the same conditions as Experimental Example 5 except that the temperature of dry gas was 300°C. The results of measurement are shown in Figure 2. It can be understood that the intensity of peak 4 resulting from 2,3,3,3-tetrafluoro-2-(1,1,2,2,3,3,3-heptafluoropropoxy)propanoic acid is lowered.

## Claims

1. A mass spectrometry method for a fluoroether compound, the method comprising:
1) preparing a sample solution containing the fluoroether compound and a solvent,
2) ionizing the fluoroether compound in the sample solution and also removing the solvent to produce an ion, and
3) mass-separating the ion to determine the mass of the ion,
wherein
in the production of the ion,
the sample solution is sprayed together with an atomizing gas at 330°C or lower to produce a droplet, and the produced droplet is brought into contact with a dry gas at 190°C or lower.

2. The mass spectrometry method according to claim 1, wherein the sample solution has a pH of less than 8.

3. The mass spectrometry method according to claim 1 or 2, wherein a pressure when producing the ion is atmospheric pressure.

4. The mass spectrometry method according to any one of claims 1 to 3, wherein the production of the ion is performed by an electrospray ionization method.

5. The mass spectrometry method according to any one of claims 1 to 4, wherein the fluoroether compound is a compound represented by the general formula: 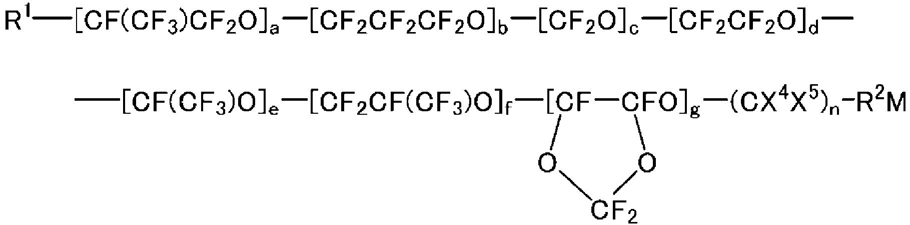 wherein R¹ is H-, F-, Cl-, CH₂=CH-, CH₂=CF-, CF₂=CF-, or CF₂=CFO-; R² is COO or SO₃; a to g are the same or different and are each an integer of 0 or more, the sum of a to g is an integer of 0 or 1 or more, and when the sum of a to g is 0, R¹ is CF₂=CFO-; X⁴ and X⁵ are each independently H, F, or CF₃; n is an integer of 1 or more; and M is a cation.

6. The mass spectrometry method according to claim 5, wherein an ion represented by the following general formula is used as a precursor ion: 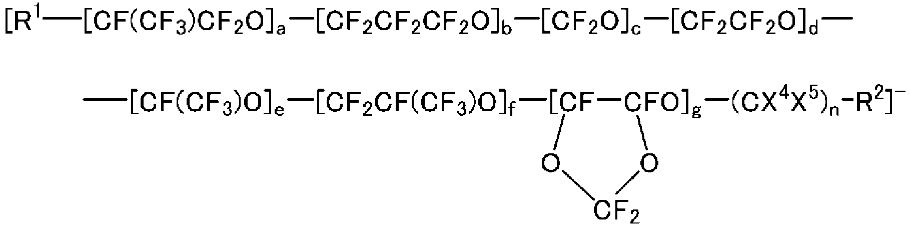 wherein R¹, R², a to g, X⁴, X⁵, and n are as described above.

7. The mass spectrometry method according to any one of claims 1 to 6, comprising preparing a sample solution further containing a fluorine-containing compound different from the fluoroether compound, ionizing the fluorine-containing compound in addition to the fluoroether compound and also removing the solvent to produce the ion of the fluoroether compound and an ion of the fluorine-containing compound, and determining the mass of the ion of the fluorine-containing compound in addition to the mass of the ion of the fluoroether compound.
